(19) Europäisches Patentamt
European Patent Office
Office européen des brevets

(11) EP 0 787 715 B1

(12) **EUROPÄISCHE PATENTSCHRIFT**

(45) Veröffentlichungstag und Bekanntmachung des Hinweises auf die Patenterteilung:
**26.01.2000 Patentblatt 2000/04**

(51) Int Cl.[7]: **C07C 213/10**

(21) Anmeldenummer: **96120726.3**

(22) Anmeldetag: **21.12.1996**

(54) **Verfahren zur Racematspaltung von Tramadol**

Process for the optical resolution of tramadol

Procédé pour la résolution optique du tramadol

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **19.01.1996 DE 19601745**

(43) Veröffentlichungstag der Anmeldung:
**06.08.1997 Patentblatt 1997/32**

(73) Patentinhaber: **Grünenthal GmbH**
**52078 Aachen (DE)**

(72) Erfinder:
- **Buschmann, Helmut, Dr.**
  **52066 Aachen (DE)**
- **Winter, Werner, Prof. Dr.**
  **52078 Aachen (DE)**
- **Graudums, Ivars, Dr.**
  **52222 Stolberg (DE)**
- **Jansen, Peter**
  **52249 Eschweiler (DE)**

(56) Entgegenhaltungen:
- **ARZNEIM.-FORSCH. (1978), 28(1A), 114-21 CODEN: ARZNAD;ISSN: 0004-4172, 1978, XP000644506 FRANKUS, E. ET AL: "Separation of isomers, structural elucidation and pharmacological characterization of 1-(m-methoxyphenyl)-2-(dimethylaminomethyl ) -1- cyclohexanol"**
- **J. JACQUES ET AL.: "Enantiomers, racemates and resolutions" 1991 , KRIEGER PUBLISHING COMPANY , MALABAR, FLORIDA (U.S.A) XP002030514 20422 * Seite 259 - Seite 261 * * Seite 387 - Seite 388 ***

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Racematspaltung von Tramadol.

Tramadolhydrochlorid - (1RS, 2RS)-2-Dimethylaminomethyl-1-(3-methoxyphenyl)-cyclohexanol, Hydrochlorid - nimmt unter den zentralwirksamen Analgetika eine Sonderstellung ein, da dieser Wirkstoff eine starke Schmerzhemmung ohne die für Opioide bekannten Nebenwirkungen hervorruft (J. Pharmacol. Exptl. Ther. 267, 331 (1993)). Tramadol ist ein Racemat und besteht aus gleichen Mengen an (+)- und (-)-Enantiomer. Es ist bekannt, daß die Enantiomere von Tramadol ein interessantes, von Tramadol abweichendes pharmakologisches Profil besitzen. Das (+)-Enantiomere zeichnet sich durch eine opiatartige analgetische Wirkung aus, die im Vergleich zu Tramadol deutlich verstärkt ist, während beim (-)-Enantiomeren eine deutliche Hemmung der Noradrenalin-Wiederaufnahme beobachtet wird.

Die Herstellung der Tramadol-Enantiomeren unter Verwendung des Racematspaltungsreagenzes Dibenzoyl-weinsäure ist in Arzneim.-Forsch./Drug Res. 28 (I), 114 (1978) beschrieben. Nachteilig an diesem Verfahren ist der Einsatz des sehr teuren chiralen Hilfsreagenzes Dibenzoyl-weinsäure. Diese Verbindung läßt sich nur mit erheblichen Schwierigkeiten erneut in der Racematspaltung einsetzen, da während der alkalischen Aufarbeitung der diastereomeren Salze die Dibenzoylgruppen teilweise abgespalten werden. Darüber hinaus werden zur Isolierung der Tramadol-Enantiomeren die optischen Antipoden der Dibenzoyl-weinsäure benötigt: Um das (+)-Enantiomere von Tramadol zu erhalten, muß mit (-)-O,O'-Dibenzoyl-L-weinsäure gefällt werden; um das (-)-Enantiomere von Tramadol zu erhalten, muß mit (+)-O,O'-Dibenzoyl-D-weinsäure gefällt werden.

Die der Erfindung zugrundeliegende Aufgabe bestand daher in der Entwicklung eines Verfahrens zur Tramadol-Racematspaltung, mit dem sich die Tramadol-Enantiomeren unter Vermeidung der bekannten, mit dem Einsatz von Dibenzoyl-weinsäure verbundenen Nachteilen in konstant hohen Ausbeuten und hohen Enantiomerenreinheiten erhalten lassen.

Überraschenderweise wurde gefunden, daß mit dem Einsatz der kostengünstigen L-(+)-Weinsäure die an das zu entwickelnde Verfahren gestellten hohen Anforderungen erfüllt werden.

Dementsprechend ist Erfindungsgegenstand ein Verfahren zur Racematspaltung von Tramadol, welches dadurch gekennzeichnet ist, daß man ein racemisches Tramadolsalz in die Base überführt, mit L-(+)-Weinsäure das (-)-Enantiomere von Tramadol durch Fällung abtrennt und aus der Mutterlauge der Weinsäurefällung das (+)-Enantiomere von Tramadol durch Freisetzung der Tramadol-Base und anschließender Überführung in ein von Tartrat verschiedenes Salz isoliert.

Für das erfindungsgemäße Verfahren eignet sich als Edukt insbesondere racemisches Tramadolhydrochlorid. Dieses wird in einer wäßrigen Lösung unter Zusatz von Alkalihydroxiden, vorzugsweise Natriumhydroxid, und Extraktion mit einem organischen Lösungsmittel, beispielsweise Dichlormethan und/oder Diethylether, in racemisches Tramadol überführt. Anschließend wird die erhaltene Base mit L-(+)-Weinsäure vorzugsweise in Gegenwart eines organischen Lösungsmittels, besonders bevorzugt in Gegenwart eines aliphatischen $C_{1-5}$-Alkohols versetzt. Das sich bildende Tartrat des (-)-Enantiomeren von Tramadol wird insbesondere durch Kristallisation von dem gebildeten Tartrat des (+)-Tramadol-Enantiomeren abgetrennt und anschließend gewünschtenfalls durch Freisetzung der Tramadol-Base unter den vorgenannten Bedingungen und Überführung in ein von Tartrat verschiedenes Tramadolsalz, vorzugsweise in Hydrochlorid, isoliert.

Das in der Mutterlauge lösliche (+)-Tramadol-Enantiomere in Form des Tartratsalzes wird durch Freisetzung der Tramadol-Base unter den vorgenannten Bedingungen und anschließende Überführung in ein Salz, das nicht Tartrat ist, insbesondere in das Tramadolhydrochlorid, isoliert.

Die Umwandlung der Tramadol-Base in das Hydrochlorid kann mit konzentrierter Salzsäure oder gasförmigem Chlorwasserstoff in einem organischen Lösungsmittel, beispielsweise Aceton, Dioxan, Diethylether und/oder Diisopropylether, oder mit Trimethylchlorsilan/Wasser in einem Lösungsmittel, beispielsweise 2-Butanon, durchgeführt werden.

Das erfindungsgemäße Verfahren läßt sich kostengünstig und umweltfreundlich durchführen. Im Vergleich zur Racematspaltung mit Dibenzoyl-weinsäure zeichnet sich das erfindungsgemäße Verfahren dadurch aus, daß nur eine enantiomere Form der Weinsäure, nämlich die kostengünstige L-(+)-Weinsäure, zur Racematspaltung erforderlich ist. Mit L-(+)-Weinsäure lassen sich die Enantiomeren bezogen auf eingesetztes Racemat in über 85%iger Ausbeute mit einer Enantiomeren-Reinheit von über 98 % erhalten. Darüber hinaus besitzt L-(+)-Weinsäure im Vergleich zur Dibenzoylweinsäure ein um den Faktor 2,4 kleineres Formelgewicht, mit der Folge, daß die anfallenden Abfallprodukte erheblich reduziert werden. Ferner kann die Mutterlauge nach Freisetzung der Tramadol-Base in den Racematspaltungsprozeß zurückgeführt werden.

## Beispiele

### Beispiel 1

(-)-(1S,2S)-2-Dimethylaminomethyl-1-(3-methoxy-phenyl)-cyclohexanol, Hydrochlorid (-1)

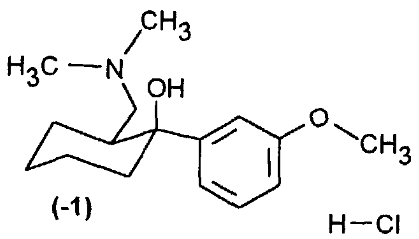

(-1)

1. Stufe: Freisetzung der racemischen Base

3 kg (10 mol) (1RS, 2RS)-2-Dimethylaminomethyl-1-(3-methoxy-phenyl)-cyclohexanol, Hydrochlorid (1) wurden in 4800 ml Wasser suspendiert und mit 1,6 kg zerstoßenem Eis versetzt. Unter Rühren wurden 1300 ml 36-38 %ige Natronlauge (technisch) zugetropft. Anschließend wurde mit 7000 ml Dichlormethan und nach Phasentrennung mit weiteren 2000 ml Dichlormethan extrahiert. Die vereinigten organischen Phasen wurden über Natriumsulfat getrocknet. Nach destillativer Entfernung des Lösungsmittels wurden 2630 g (99 % der Theorie) (1RS, 2RS)-2-Dimethylaminomethyl-1-(3-methoxy-phenyl)-cyclohexanol als Sirup erhalten.

2. Stufe: Fällung mit L-(+)-Weinsäure

2630 g (10 mol) der Base aus der ersten Stufe wurden in 2400 ml Ethanol gelöst und mit einer Lösung bestehend aus 1500 g (10 mol) L-(+)-Weinsäure und 11200 ml Ethanol versetzt. Zur Kristallisation wurde zwei Stunden bei Raumtemperatur gerührt und 24 Stunden bei 4° C stehen gelassen. Die ausgefallenen Kristalle wurden abgesaugt und mit 6400 ml Ethanol von 4° C gewaschen. Nach Trocknung des Kristallisates bei Raumtemperatur im Vakuum (60 mbar) wurden 2050 g (49 % % bezogen auf die Gesamtmenge an eingesetzter racemischer Base) (1S, 2S)-2-Dimethylaminomethyl-1-(3-methoxy-phenyl)-cyclohexanol-L-(+)-tartrat mit einem Schmelzpunkt von 173-175° C erhalten (Drehwert: $[\alpha]_D^{RT}$ = -12,2° (c = 1,01; Methanol).

3. Stufe: Freisetzen der Base aus dem L-(+)-Weinsäuresalz

2050 g (4,95 mol) (1S, 2S)-2-Dimethylaminomethyl-1-(3-methoxy-phenyl)-cyclohexanol-L-(+)-tartrat aus Stufe 2 wurden in 4000 ml Wasser gelöst und mit 900 g zerstoßenem Eis versetzt. Unter Rühren wurden 1000 ml 36-38 %ige Natronlauge (technisch) zugetropft. Anschließend wurde mit 2500 ml Dichlormethan und nach Phasentrennung mit weiteren 500 ml Dichlormethan extrahiert. Die vereinigten organischen Phasen wurden über Natriumsulfat getrocknet. Nach destillativer Entfernung des Lösungsmittels wurden 1280 g (99 % der Theorie) (1S, 2S)-2-Dimethylaminomethyl-1-(3-methoxy-phenyl)-cyclohexanol als Sirup erhalten.

4. Stufe: Überführung von (1S, 2S)-2-Dimethylaminomethyl-1-(3-methoxy-phenyl)-cyclohexanol in das Hydrochlorid (-1)

1280 g (4,86 mol) der aus Stufe 3 erhaltenen Base wurden in 16 l 2-Butanon gelöst und unter Rühren mit 88 ml (4,9 mol) Wasser sowie 621 ml (532 g; 4,9 mol) Trimethylchlorsilan versetzt. Zur Kristallisation wurde 3 Stunden bei Raumtemperatur gerührt und 24 Stunden bei 4° C stehen gelassen. Der ausgefallene Feststoff wurde abgesaugt, mit 5000 ml 2-Butanon von 4° C gewaschen und bei 90° C im Vakuum (60 mbar) bis zur Gewichtskonstanz getrocknet. Es wurden 1390 g (95 % der Theorie bezogen auf die eingesetzte Base aus Stufe 3 und 92 % bezogen auf den Enantiomerenanteil des eingesetzten Racemates aus Stufe 1) Hydrochlorid (-1) als farblose Kristalle erhalten.

Schmelzpunkt: 172-173° C
Drehwert: $[\alpha]_D^{RT}$ = -29,6° (c = 1,00; Methanol)

**Beispiel 2**

(+)-(1R, 2R)-2-Dimethylaminomethyl-1-(3-methoxy-phenyl)-cyclohexanol, Hydrochlorid (+1)

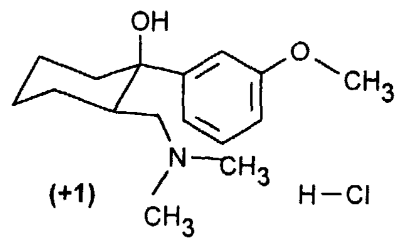

(+1)

1. Stufe: Freisetzen der Base aus der Mutterlauge der L-(+)-Weinsäure-Fällung

Die ethanolische Mutterlauge sowie die Waschphase aus der L-(+)-Weinsäurefällung (Beispiel 1, 2. Stufe) wurden vereinigt. Nach destillativer Entfernung des Lösungsmittels wurde der Rückstand (2080 g) in 2500 ml Wasser gelöst und mit 900 g zerstoßenem Eis versetzt. Unter Rühren wurden 1000 ml 36-38 %ige Natronlauge (technisch) zugetropft. Anschließend wurde mit 2700 ml Dichlormethan und nach Phasentrennung mit weiteren 600 ml Dichlormethan extrahiert. Die vereinigten organischen Phasen wurden über Natriumsulfat getrocknet. Nach destillativer Entfernung des Lösungsmittels wurden 1340 g (99 % der Theorie) (1R, 2R)-2-Dimethylaminomethyl-1-(3-methoxy-phenyl)-cyclohexanol als Sirup erhalten.

2. Stufe: Überführung von (1R, 2R)-2-Dimethylamino-methyl-1-(3-methoxy-phenyl)-cyclohexanol in das Hydrochlorid (+1)

1340 g (5,09 mol) der aus Stufe 1 erhaltenen Base wurden in 17,5 l 2-Butanon gelöst und unter Rühren mit 105 ml (5,8 mol) Wasser sowie 670 ml (573 g; 5,3 mol) Trimethylchlorsilan versetzt. Zur Kristallisation wurde 3 Stunden bei Raumtemperatur gerührt und 24 Stunden bei dieser Temperatur stehen gelassen. Der ausgefallene Feststoff wurde abgesaugt, mit 5000 ml 2-Butanon gewaschen und bei 90° C im Vakuum (60 mbar) bis zur Gewichtskonstanz getrocknet. Es wurden 1350 g (88 % der Theorie bezogen auf die eingesetzte Base aus Stufe 1 und 89 % bezogen auf den Enantiomerenanteil des eingesetzten Racemates aus Beispiel 1, Stufe 1) Hydrochlorid (+1) als farblose Kristalle erhalten.

| | |
|---|---|
| Schmelzpunkt: | 171-172° C |
| Drehwert: | $[\alpha]_D^{RT}$ = +29,6° (c = 1,00; Methanol) |

**Patentansprüche**

1. Verfahren zur Racematspaltung von Tramadol, dadurch gekennzeichnet, daß man ein racemisches Tramadolsalz in die Base überführt, mit L-(+)-Weinsäure das (-)-Tramadol-Enantiomere durch Fällung abtrennt und aus der Mutterlauge der Weinsäurefällung das (+)-Tramadol-Enantiomere durch Freisetzung der Base und anschließender Überführung in ein von Tartrat verschiedenes Salz isoliert.

2. Verfahren nach Anspruch 1, dadurch gekennzeichnet, daß racemisches Tramadolhydrochlorid eingesetzt wird.

3. Verfahren nach einem oder beiden der Ansprüche 1 bis 2, dadurch gekennzeichnet, daß L-(+)-Weinsäure in Gegenwart eines organischen Lösungsmittels, vorzugsweise in Gegenwart eines aliphatischen $C_{1-5}$-Alkohols eingesetzt wird.

4. Verfahren nach einem oder mehreren der Ansprüche 1 bis 3, dadurch gekennzeichnet, daß das (-)-Tramadol-Enantiomere durch Kristallisation abgetrennt wird.

5. Verfahren nach einem oder mehreren der Ansprüche 1 bis 4, dadurch gekennzeichnet, daß das (-)- und das (+)-Tramadol-Enantiomere als Hydrochlorid isoliert wird.

**Claims**

1. Process for resolving racemic mixtures of tramadol, characterised in that a racemic tramadol salt is converted into the base, the (-) tramadol enantiomer is separated by precipitation with L-(+)-tartaric acid and the (+) tramadol enantiomer is isolated from the tartaric acid precipitation mother liquor by liberation of the base and subsequent conversion into a salt differing from tartrate.

2. Process according to claim 1, characterised in that racemic tramadol hydrochloride is used.

3. Process according to one or both of claims 1 to 2, characterised in that L-(+)-tartaric acid is used in the presence of an organic solvent, preferably in the presence of an aliphatic $C_{1-5}$ alcohol.

4. Process according to one or more of claims 1 to 3, characterised in that the (-) tramadol enantiomer is separated by crystallisation.

5. Process according to one or more of claims 1 to 4, characterised in that the (-) and (+) tramadol enantiomer is isolated as the hydrochloride.

**Revendications**

1. Procédé de résolution optique du racémate du tramadol, caractérisé en ce qu'on transforme un sel racémique de tramadol en la base, on sépare l'énantiomère(-) du tramadol par précipitation avec l'acide L-(+)-tartrique et on isole de la liqueur-mère de précipitation par l'acide tartrique l'énantiomère (+) du tramadol par libération de la base puis transformation en un sel différent du tartrate.

2. Procédé suivant la revendication 1, caractérisé en ce qu'on utilise du chlorhydrate de tramadol racémique.

3. Procédé suivant l'une des revendications 1 et 2 ou les deux, caractérisé en ce qu'on utilise l'acide L-(+)-tartrique en présence d'un solvant organique, de préférence en présence d'un alcool aliphatique en $C_1$ à $C_5$.

4. Procédé suivant une ou plusieurs des revendications 1 à 3, caractérisé en ce que l'énantiomère(-) du tramadol est séparé par cristallisation.

5. Procédé suivant une ou plusieurs des revendications 1 à 4, caractérisé en ce qu'on isole l'énantiomère(-) et l'énantiomère(+) du tramadol sous forme de chlorhydrate.